## Europäisches Patentamt

## European Patent Office

## Office européen des brevets

(19)

(11) Veröffentlichungsnummer: **0 090 283**
**B1**

(12) **EUROPÄISCHE PATENTSCHRIFT**

(45) Veröffentlichungstag der Patentschrift:
04.12.85

(21) Anmeldenummer: 83102672.9

(22) Anmeldetag: 18.03.83

(51) Int. Cl.⁴: **C 07 C 11/02**, C 07 C 1/20,
B 01 J 29/04

(54) Verfahren zur Herstellung von Olefinen aus Methanol/Dimethylether.

(30) Priorität: 27.03.82 DE 3211399
14.01.83 DE 3300982

(43) Veröffentlichungstag der Anmeldung:
05.10.83 Patentblatt 83/40

(45) Bekanntmachung des Hinweises auf die Patenterteilung:
04.12.85 Patentblatt 85/49

(84) Benannte Vertragsstaaten:
BE DE FR GB NL

(56) Entgegenhaltungen:
EP - A - 0 007 081
US - A - 4 292 458

(73) Patentinhaber: BASF Aktiengesellschaft,
Carl-Bosch-Strasse 38, D-6700 Ludwigshafen (DE)

(72) Erfinder: Hoelderich, Wolfgang, Dr., Mannheimer
Strasse 18 C, D-6710 Frankenthal (DE)
Erfinder: Himmel, Walter, Dr., Hardtstrasse 3,
D-6701 Fussgoenheim (DE)
Erfinder: Mross, Wolf Dieter, Dr.,
Anselm-Feuerbach-Strasse 21, D-6710 Frankenthal (DE)
Erfinder: Schwarzmann, Matthias, Dr.,
Carl-Bosch-Strasse 54, D-6703 Limburgerhof (DE)

## Beschreibung

In neuerer Zeit gewinnen Bemühungen, Methanol zur Herstellung von Olefinen zu verwenden, zunehmendes Interesse. Methanol läßt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff weiter beibehalten werden. In den vergangenen Jahren sind daher Verfahren entwickelt worden, die die Herstellung von Olefinen aus Methanol und/oder Dimethylether zum Gegenstand haben. Ein solches Verfahren ist beispielsweise in der DE-OS 2 615 150 beschrieben. Als Katalysator wird dabei der Aluminosilikatzeolith ZSM-5 verwendet, der eigentlich ein Aromatisierungskatalysator ist. Die Umsetzung kann aber durch verschiedene Maßnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethylether mit Inertgasen bzw. Wasserdampf oder die Verdünnung des Katalysators mit Bindemitteln. Die Erfahrung zeigt, daß hohe Olefinausbeuten nur durch eine sehr starke Verdünnung von Methanol und/oder Dimethylether mit Inertgas oder Wasserdampf zu erzielen sind. Andere bekanntgewordene Verfahren haben als Nachteil eine geringe Belastbarkeit und eine schnelle Verkokung des Katalysators. Es besteht aber großes Interesse an einem einfachen Verfahren, das die vollständige Umsetzung von Rohmethanol und/oder Dimethylether in überwiegend aus $C_2-C_4$-Olefinen bestehende Kohlenwasserstoffe ermöglicht.

Es wurde nun gefunden, daß man $C_2-C_4$-Olefinen in hoher Ausbeute aus Methanol und/oder Dimethylether durch katalytische Umwandlung bei erhöhter Temperatur in Gegenwart von Zeolithkatalysatoren erhält, wenn man als Katalysatoren Borosilikatzeolithe verwendet, die als Bindemittel amorphe Aluminiumsilikate enthalten.

Vorteilhaft wendet man Katalysatoren an, bei denen im Bindemittel ein $SiO_2/Al_2O_3$-Verhältnis von 35 : 65 bis 75 : 25 Gew.-% eingehalten wird.

Der Erfindung lag die Aufgabe zugrunde, die Anteile an höheren aliphatischen Kohlenwasserstoffen zugunsten der $C_2-C_4$-Olefine zu verringern, ohne die Gesamtkohlenwasserstoffausbeute herabzusetzen. Dies wird dadurch erreicht, daß man zu den C—C verknüpfenden Reaktionen konkurrierende und simultan verlaufende Crackreaktionen einleitet, die selektiv zu den gewünschten $C_2-C_4$-Olefinen abbauen.

Methanol und/oder Dimethylether werden an den mit amorphen Aluminiumsilikaten verstrangten Borosilikatzeolithen bei einem Druck zwischen Normaldruck und ca. 30 bar und bei Temperaturen zwischen 300°C und 700°C, bevorzugt zwischen 400°C und 550°C, umgesetzt. Das Methanol kann einen Wassergehalt bis zu 90 Gew.-% haben. Bevorzugt wird Rohmethanol, wie es aus der Methanolsynthese kommt und das ca. 20 Gew.-% Wasser enthält, eingesetzt. Dem Methanol können auch noch andere niedere Alkohole beigemischt sein. Die Belastung des Katalysators, ausgedrückt in WHSV = $h^{-1}$, d. h. g Methanol und/oder Dimethylether pro g Katalysator und Stunde, wird vorteilhafterweise so gewählt, daß diese Einsatzstoffe möglichst quantitativ umgesetzt werden, damit keine Abtrenn- und Rückführprobleme entstehen. Im allgemeinen wird daher WHSV im Bereich von 0,5 bis 50 $h^{-1}$, vorzugsweise von 2 bis 15 $h^{-1}$, liegen. Ein besonderer Vorteil der Erfindung ist es, daß man die Umsetzung von Rohmethanol bzw. Dimethylether in $C_2$- bis $C_4$-Olefine ohne Verdünnungsmittel an den oben beschriebenen Bor-Zeolithkatalysatoren ausführen kann.

Die Durchführung des erfindungsgemäßen Verfahrens wird anhand der nachstehenden Beispiele näher erläutert.

### Herstellung des Katalysators

Der Bor-Zeolith wird in einer hydrothermalen Synthese aus 64 g $SiO_2$ (Aerosil 200), 12,2 g $H_3BO_3$, 800 g einer wäßrigen 1,6-Hexandiamin-Lösung (Mischung 50 : 50) bei 170°C unter autogenem Druck in einem Rührautoklaven hergestellt. Nach Abfiltrieren und Auswaschen wird das kristalline Reaktionsprodukt bei 160°C 24 h getrocknet und bei 500°C 24 h calciniert. Dieser Borosilikatzeolith setzt sich zusammen aus 94,2 Gew.-% $SiO_2$ und 2,32 Gew.-% $B_2O_3$.

Aus diesem Borzeolith werden Katalysatoren mit verschiedenem Bindemittelzusatz hergestellt:

Katalysator A wird erhalten durch die Verstrangung von 72 g des oben beschriebenen Borosilikatzeolithen mit 48 g eines amorphen Aluminosilikates, das sich aus 65% $Al_2O_3$ und 35% $SiO_2$ zusammensetzt. Der Preßdruck beträgt ca. 100 bar. Die 2-mm-Stränge werden bei 110°C 16 h getrocknet und bei 500°C 16 h calciniert.

Katalysator B wird wie Katalysator A erhalten. Das eingesetzte amorphe Aluminosilikat setzt sich aber aus 45% $Al_2O_3$ und 55% $SiO_2$ zusammen.

Katalysator C wird wie Katalysator A erhalten. Das eingesetzte amorphe Aluminosilikat setzt sich aber aus 25% $Al_2O_3$ und 75% $SiO_2$ zusammen.

Vergleichskatalysator D wird erhalten durch Verstrangung des oben beschriebenen Borosilikatzeolithen mit Boehmit im Verhältnis 60 : 40. Die Trocknung erfolgt bei 110°C 16 h und die Calcinierung bei 500°C 16 h. Dieser Katalysator D dient als Vergleichskatalysator; Boehmit besitzt keine Crackeigenschaften.

Beispiel

An den Katalysatoren A bis D wird unter isothermen Bedingungen in einem Rohrreaktor Rohmethanol (20% Wasser) bei 500°C und WHSV = 7,6 h$^{-1}$ quantitativ umgesetzt. Die Ausbeuten an Kohlenwasserstoffen sind in der Tabelle angegeben.

Tabelle

|  | Katalysator | | | |
|---|---|---|---|---|
|  | A | B | C | D |
| $CH_4$ % | 4,2 | 4,8 | 4,5 | 5,5 |
| $C_2H_4$ % | 12,2 | 11,3 | 10,9 | 7,9 |
| $C_2H_6$ % | 0,4 | 0,4 | 0,4 | — |
| $C_3H_6$ % | 32,8 | 33,4 | 34,2 | 30,4 |
| $C_3H_8$ % | 2,1 | 1,4 | 1,4 | 1,6 |
| $C_4$ % | 20,5 | 20,3 | 20,8 | 16,8 |
| $C_{5+}$ % | 25,5 | 26,2 | 25,5 | 34,5 |
| Laufzeit h*) | 14 | 14 | 17 | 10 |
| g $CH_3OH$/g Kontakt | 109 | 109 | 133 | 78 |

*) Laufzeit bis zur 1. Regenerierung

**Patentansprüche**

1. Verfahren zur Herstellung von Olefinen durch Umsetzung von Methanol und/oder Dimethylether in Gegenwart von Zeolithkatalysatoren bei erhöhter Temperatur, dadurch gekennzeichnet, daß man als Katalysatoren Borosilikatzeolithe verwendet, die als Bindemittel amorphe Aluminiumsilikate enthalten.

2. Verfahren nach Anspruch 1, dadurch gekennzeichnet, daß man Katalysatoren anwendet, bei denen im Bindemittel ein $SiO_2/Al_2O_3$-Verhältnis von 35 : 65 bis 75 : 25 Gew.-% eingehalten wird.

**Claims**

1. A process for the preparation of olefins by reacting methanol and/or dimethyl ether in the presence of a zeolitic catalyst at elevated temperature, wherein a borosilicate zeolite, containing an amorphous aluminosilicate as binder, is used as the catalyst.

2. A process as claimed in claim 1, wherein a catalyst is used in which a ratio of $SiO_2$ to $Al_2O_3$, in percent by weight, of 35 : 65 to 75 : 25 is maintained in the binder.

**Revendications**

1. Procédé pour la préparation d'oléfines par transformation de méthanol et/ou de diméthyléther en présence de catalyseurs zéolitiques à température élevée, caractérisé en ce qu'on utilise comme catalyseurs des zéolites de borosilicate qui contiennent, en tant que liant, des silicates d'aluminium amorphes.

2. Procédé selon la revendications 1, caractérisé en ce qu'on utilise des catalyseurs dans lesquels est maintenu, dans le liant, un rapport $SiO_2/Al_2O_3$ de 35 : 65 à 75 : 25.